(19) Europäisches Patentamt
European Patent Office
Office européen des brevets

(11) Veröffentlichungsnummer: **0 030 518**
**A1**

(12) # EUROPÄISCHE PATENTANMELDUNG

(21) Anmeldenummer: **80810334.5**

(51) Int. Cl.³: **C 07 C 127/22**, A 01 N 47/34

(22) Anmeldetag: **03.11.80**

(30) Priorität: **08.11.79 CH 10002/79**
**03.09.80 CH 6627/80**

(43) Veröffentlichungstag der Anmeldung: **17.06.81**
**Patentblatt 81/24**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

(54) **Substituierte N-3-Trifluormethyl-N-benzoylharnstoffe, Verfahren zu ihrer Herstellung, diese Verbindungen enthaltende Mittel und ihre Verwendung zur Bekämpfung von Schädlingen.**

(57) Neue substituierte N-3-Trifluormethylphenyl-N'-benzoylharnstoffe der Formel

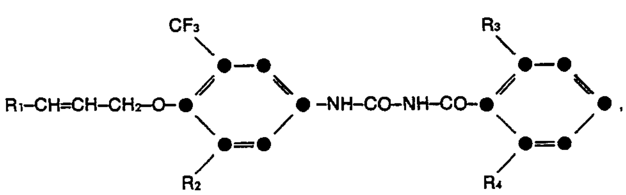

worin $R_1$ Wasserstoff, Chlor oder Brom; $R_2$ Chlor oder Brom; $R_3$ Fluor oder Chlor; und $R_4$ Wasserstoff, Fluor oder Chlor bedeuten, Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen sind insbesondere wirksam gegen larvale Stadien fressender, pflanzenschädigender Insekten.

EP 0 030 518 A1

ACTORUM AG

0030518



CIBA-GEIGY AG
BASEL (Schweiz)

5-12592/1+2

BEZEICHNUNG GEÄNDERT
siehe Titelseite

Phenylharnstoffe

Die vorliegende Erfindung betrifft neue substituierte N-3-Trifluormethylphenyl-N'-benzoylharnstoffe, Verfahren zu ihrer Herstellung und ihre Verwendung in der Schädlingsbekämpfung.

Die erfindungsgemässen substituierten N-3-Trifluormethylphenyl-N'-benzoylharnstoffe haben die Formel I

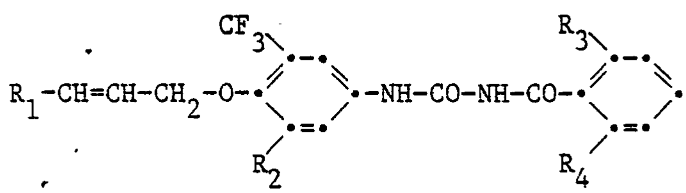 (I) ,

worin $R_1$ Wasserstoff, Chlor oder Brom; $R_2$ Chlor oder Brom; $R_3$ Fluor oder Chlor; und $R_4$ Wasserstoff, Fluor oder Chlor bedeuten.

Wegen ihrer Wirkung als Schädlingsbekämpfungsmittel bevorzugt sind erfindungsgemässe Verbindungen der Formel I, die dadurch gekennzeichnet sind, dass $R_1$ und $R_2$ Chlor oder Brom und $R_3$ sowie $R_4$ Fluor bedeuten. Weiterhin bevorzugt sind Verbindungen der Formel I, worin $R_1$ und $R_2$ Chlor oder Brom, $R_3$ Chlor und $R_4$ Wasserstoff bedeuten. Hervorzuheben sind auch Verbindungen der Formel I, worin $R_1$ Chlor bedeutet.

Die Verbindungen der Formel I fallen, wo dies prinzipiell möglich ist, als cis-trans Isomerengemische an. In diesem Sinne sind als Verbindungen der Formel I sowohl die cis- oder trans-Formen als

auch die entsprechenden Isomerengemische zu verstehen. Ein Isomerengemisch kann z.B. mit Hilfe der bekannten chromatographischen Trennmethoden und anschliessender Eluierung in die isomeren Formen getrennt werden. Zur Synthese stereochemisch einheitlicher Verbindungen der Formel I verwendet man mit Vorteil stereochemisch einheitliche Ausgangsverbindungen der nachstehnden Formeln II bzw. IV.

Die Verbindungen der Formel I können nach an sich bekannten Verfahren hergestellt werden (vgl. u.a. die deutschen Offenlegungsschriften Nr. 2.123.236 und 2.601.780).

So kann man z.B. eine Verbindung der Formel I erhalten durch Umsetzung

a) einer Verbindung der Formel II

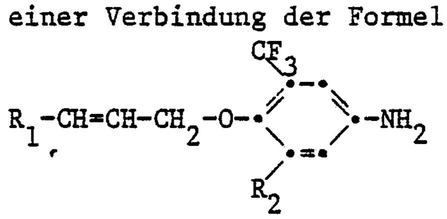

(II)

mit einer Verbindung der Formel III

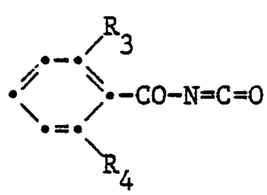

(III)

oder

b) einer Verbindung der Formel IV

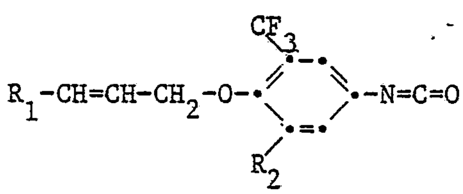

(IV)

gegebenenfalls in Gegenwart einer organischen oder anorganischen Base

mit einer Verbindung der Formel V

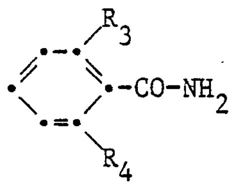

(V).

In den obigen Formeln II, III, IV und V haben die Reste $R_1$, $R_2$, $R_3$ und $R_4$ die unter Formel I vorstehend angegebenen Bedeutungen.

Die erwähnten Verfahren a) und b) können vorzugsweise unter normalem Druck und in Gegenwart eines inerten organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Methyläthylketon, Methylisopropylketon und Methylisobutylketon. Verfahren a) wird im allgemeinen bei einer Temperatur von -10 bis 100°C, vorzugsweise zwischen 0 und 25°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden. So sind die substituierten Aniline der Formel II gemäss literaturbekannten Verfahren herstellbar (vgl. europäische Patentanmeldung 0000542), indem man z.B. 2-Trifluormethyl-6-chlor-4-nitrophenol in Anwesenheit von Acetanhydrid katalytisch hydriert und das entstandene 2-Trifluormethyl-6-chlor-4-acetaminophenol mit den entsprechend substituierten Alkenylbromiden oder -chloriden veräthert.

Anschliessend wird die N-Acetylgruppe in üblicher Weise (z.B. mit wässriger Salzsäure in Alkohol) abgespalten. Einige der Aniline der Formel II sind ferner durch chemische Reduktion (z.B. mittels Sn-(II)-Chlorid/HCl) entsprechender p-Nitro-Phenyläther der Formel

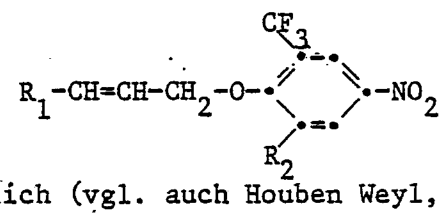

zugänglich (vgl. auch Houben Weyl, "Methoden d.org. Chemie" 11/1, 422).

Zu den Benzoylisocyanaten der Formel III kann man unter anderem wie folgt gelangen (vgl. J.Agr.Food Chem. 21, 348 und 993, 1973):

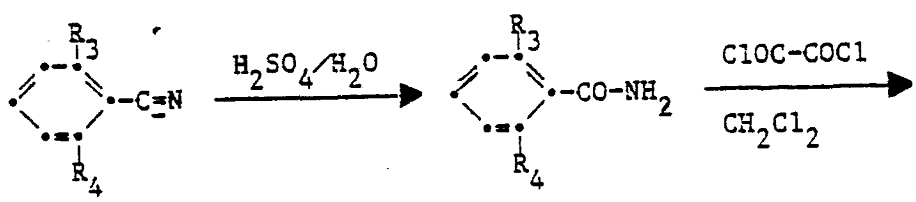


(III).

Die substituierten Phenylisocyanate der Formel IV lassen sich z.B. durch Phosgenisierung der entsprechenden Aniline der Formel II nach allgemein üblichen Verfahren herstellen. Die weiterhin als Ausgangsstoffe zu verwendenden Benzamide der Formel V sind zumeist bekannt (vgl. Beistein "Handbuch der organischen Chemie" Bd. 9, S. 336).

Es ist bereits bekannt, dass bestimmte N-Phenyl-N'-benzoylharnstoffe insektizide Eigenschaften besitzen (vgl. deutsche Offenlegungsschriften 2.123.236, 2.504.982 und 2.537.413, die belgische Patentschriften 832.304, 843,906, und 844.066 sowie die US-Patentschriften

4.089.975 und 4.139.636). Halogensubstituierte N-3-Trifluormethylphenyl-N'-benzoyl-harnstoffe mit insektizider Wirkung sind aus der US-Patentschrift 4.085.226 und J.Agr. Food Chem. 21, 348 (1973) bekannt. Weiterhin werden in den deutschen Offenlegungsschriften 2.601.780, 2.801.316 und 2.820.696 insektizid wirksame halogensubstituierte N-Halogenalkoxyphenyl-N'-benzoylharnstoffe und in der europäischen Patentanmeldung 0001203 sowie in der deutschen Offenlegungsschrift 2 726 684 insektizide halogensubstituierte N-Halogenalkenyl-oxyphenyl-N'-benzoylharnstoffe beschrieben.

Ueberraschenderweise wurde nun gefunden, dass die erfindungsgemässen N-(3-Trifluormethyl-4-halogenalkenyloxy-phenyl)-N'-benzoyl-harnstoffe der Formel I bei guter Pflanzenverträglichkeit und geringer Warmblütertoxität höhere Wirksamkeit bei der Bekämpfung von Pflanzen und Tiere befallenden Schädlingen, vor allem gegen Insektenlarven, aufweisen als die vorerwähnten, aus dem Stand der Technik bekannten Verbindungen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Die Verbindungen der Formel I eignen sich vor allem zur Bekämpfung von pflanzenschädigenden Frassinsekten in Zier- und Nutzpflanzungen, insbesondere in Baumwollkulturen ( z.B. Spodoptera littoralis und Heliothis virescens) sowie in Gemüsekulturen (z.B. Leptinotarsa decemlineata). Die Verbindungen der Formel I zeichnen sich durch eine ausgeprägte Wirkung gegen larvale Insektenstadien, insbesondere larvale Stadien fressender Schadinsekten, aus.

Die Verbindungen der Formel I können ferner zur Bekämpfung

von Ektoparasiten, wie Lucilia sericata, an Haus- und Nutztieren eingesetzt werden, z.B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:

Organische Phosphorverbindungen, Nitrophenole und Derivate, Formamidine, Harnstoffe, Carbamate, Pyrethroide und chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Träger und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind "cattle dips", d.h. Viehbäder, und "spray races", d.h. Sprühgänge, in denen wässrige Zubereitungen verwendet werden, zu erwähnen. Diese

Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden.

Feste Aufarbeitungsformen:

Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate; Spritzpulver (wettable powders), Pasten, Emulsionen;

b) Lösungen.

Der Gehalt an Wirkstoff in den oben beschriebenen Mitteln liegt im allgemeinen zwischen 0,1 bis 95 Gew.-%.

Die Wirkstoffe der Formel I können beispielsweise wie folgt formuliert werden.

Stäubemittel: Zur Herstellung eines a) 5 %igen und b) 2 %igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5 %igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngrösse 0,3-0,8 mm).

Die Aktivsubstanz wird mit dem epoxydierten Pflanzenöl vermischt und mit 6 Teilen Aceton gelöst, hierauf werden Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25 %igen d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25,0 Teile Wirkstoff,
4,5 Teile Calcium-Ligninsulfonat,
1,9 Teile Champagne-Kreide/Hydroxyäthylcellulose-Gemische (1:1),

1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46,0 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

<u>Emulgierbare Konzentrate</u>: Zur Herstellung eines a) 10 %igen b) 25%-igen und c) 50 %igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol;

c) 50,0 Teile Wirkstoff,
4,2 Teile Tributylphenol-Polyglykoläther,
5,8 Teile Calcium-Dodecylbenzolsulfonat,
20,0 Teile Cyclohexanon,
20,0 Teile Xylol.

Aus solchen Konzentrationen können durch Verdünnen mit Wasser Emulsionen jeder gewünschten Konzentration hergestellt werden.

Sprühmittel: Zur Herstellung eines a) 5 %igen und b) 95 %igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);

b) 95 Teile Wirkstoff,
5 Teile epoxydiertes Pflanzenöl.

Beispiel 1: Zu einer Lösung von 7,0 g 3-Trifluormethyl-4-(3-Chlorprop-2-en-1-yl)-oxy-5-brom-anilin in 20 ml abs. Aether werden bei Raumtemperatur unter Ausschluss von Feuchtigkeit 3,8 g 2,6-Difluorbenzoylisocyanat zugesetzt. Der ausgefallene Niederschlag wird nach 1 Stunde abgesaugt und mit Aether nachgewaschen. Durch Umkristallisation aus Toluol erhält man N-[3-Trifluormethyl-4-(3-chlorprop-2-en-1-yl)-oxy-5-brom]-phenyl-N'-2,6-difluorbenzoylharnstoff vom Schmelzpunkt 158 - 162°C.

Analog den vorstehend beschriebenen Arbeitsweisen werden die folgenden Verbindungen der Formel I hergestellt:

| $R_1$ | $R_2$ | $R_3$ | $R_4$ | Schmelzpunkt [°C] |
|---|---|---|---|---|
| Cl | Br | F | F | 158 - 162 *) |
| Cl | Br | Cl | H | 153 - 155 *) |
| Cl | Br | Cl | Cl | 181 - 183 *) |
| Cl | Br | F | H | 158 - 160 *) |
| H | Br | F | F | 166 - 167 |
| H | Br | F | Cl | |
| H | Br | Cl | Cl | |
| H | Br | Cl | H | 164 - 165 |
| H | Br | F | H | 144 - 146 |
| Br | Br | F | F | 129 - 142 *) |
| Br | Br | F | Cl | *) |
| Br | Br | Cl | Cl | *) |
| Br | Br | Cl | H | 141 - 146 *) |
| Br | Br | F | H | 152 - 154 *) |
| Cl | Br | F | Cl | 163 - 167 *) |
| H | Cl | F | F | |
| H | Cl | F | Cl | |
| H | Cl | Cl | Cl | |
| H | Cl | Cl | H | |
| H | Cl | F | H | |
| Cl | Cl | F | F | 166 - 167 *) |
| Cl | Cl | F | Cl | *) |
| Cl | Cl | Cl | Cl | *) |
| Cl | Cl | Cl | H | 164 - 165 *) |
| Cl | Cl | F | H | 165 - 167 *) |
| Br | Cl | F | F | 150 - 160 *) |
| Br | Cl | F | Cl | *) |

*) Stereoisomerengemisch

| $R_1$ | $R_2$ | $R_3$ | $R_4$ | Schmelzpunkt [°C] |
|---|---|---|---|---|
| Br | Cl | Cl | Cl | *) |
| Br | Cl | Cl | H | 146 - 155 |
| Br | Cl | F | H | 155 - 158 *) |

*) Stereoisomerengemisch

Beispiel 2: Wirkung gegen Musca domestica: Je 50 g frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

Beispiel 3: Wirkung gegen Lucilia sericata: Zu 9 ml eines Zuchtmediums wurden bei 50°C 1 ml einer 0,5 % Aktivsubstanz enthaltenden wässrigen Lösung gegeben. Nun wurden ca. 30 g frisch geschlüpfte Lucilia sericata-Larven zum Zuchtmedium gegeben, und nach 48, 72 und 96 Stunden wurde die insektizide Wirkung durch Ermittlung der Abtötungsrate festgestellt.

Verbindungen gemäss Beispiel 1 zeigten in diesem Test gute Wirkung gegen Lucilia sericata.

Beispiel 4: Wirkung gegen Aëdes aegypti: Auf die Oberfläche von 150 ml Wasser, das sich in einem Behälter befindet, wurde so viel einer 0,1 %igen acetonischen Lösung des Wirkstoffes pipettiert, dass Konzentrationen von je 10, 5 und 1 ppm erhalten wurden. Nach Verdunsten des Acetons wurde der Behälter mit 30 - 40 2-tägigen Aëdes-Larven beschickt. Nach 1, 2 und 5 Tagen wurde die Moralität geprüft.

Verbindungen gemäss Beispiel 1 zeigten in diesem Test gute Wirkung gegen Aëdes aegypti.

Beispiel 5: Insektizide Wirkung gegen fressende Insekten: Baumwollpflanzen wurden mit einer 0,05 %igen wässrigen Wirkstoffemulsion besprüht. Nach dem Antrocknen des Belages wurden die Baumwollpflanzen je mit Spodoptera littoralis- und Heliothis virescens-Larven im dritten larvalen Stadium besetzt. Der Versuch wurde bei 28°C und 60 % relativer Luftfeuchtigkeit durchgeführt. In Abständen von jeweils 24 Stunden wurden Mortalität sowie Entwicklungs- und Häutungsstörungen der angesetzten Larven bestimmt.

Verbindungen gemäss Beispiel 1 zeigten im obigen Test gute insektizide Wirkung gegen Spodoptera- und Heliothis-Larven.

Beispiel 6: Wirkung gegen Epilachna varivestis: Etwa 15 - 20 cm hohe Phaseolus vulgaris-Pflanzen (Buschbohnen) wurden mit einer wässrigen; den zu prüfenden Wirkstoff enthaltenden Emulsions-Zubereitung besprüht. Nach dem Antrocknen des Sprühbelages wurden pro Pflanze 10 Larven von Epilachna varivestis (Mexikanischer Bohnenkäfer) im 4. larvalen Stadium angesetzt. Ueber die behandelten

Pflanzen wurde ein Pflastikzylinder gestülpt, der mit einem Kupfer-Gazedeckel abgedeckt war.

Nach 1 und 2 Tagen wurde die akute Wirkung (% Mortalität) bestimmt. Zur Auswertung hinsichtlich allfälligem Frass-Schaden, (Antifeeding-Effekt), Entwicklungs- und Häutungsstörungen wurden die Versuchstiere während weiterer 3 Tage beobachtet.

Verbindungen gemäss Beispiel 1 zeigten gute Wirkung in obigem Test.

Beispiel 7: Wirkung gegen Leptinotarsa decemlineata (Larven): 15 cm hohe in Kulturgefässen befindliche Kartoffelpflanzen, wurden mit einer wässerigen Emulsions-Zubereitung enthaltend den zu prüfenden Wirkstoff in einer Konzentration von 500 ppm, gleichmässig bis zur Tropfnässe mit einer Druckluftspritze besprüht. Nach dem Trocknen der Pflanzen, d.h. nach etwa 1,5 Stunden, wurde über dieselben ein Plastikzylinder gestülpt, und pro Pflanze wurden je 10 Kartoffelkäferlarven des 3. Stadiums angesetzt. Die Zylinder wurden dann mit einem Kupfergaze-Deckel abgeschlossen und in der Dunkelheit bei 28°C und 60 % rel. Luftfeuchtigkeit stehen gelassen.

In Abständen von jeweils 24 Stunden wurde die Mortalität und der prozentuale Frass-Schaden an den Pflanzen geprüft.

Verbindungen gemäss Beispiel 1 zeigten gute Wirkung in obigem Test.

Beispiel 8: Chemosterilisierende Wirkung auf Anthonomus grandis. Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z.B. "Actamer B 100") desinfiziert und dann in Schalen, die eine geeignete Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 zeigten gute Wirkung im obigem Test.

Patentansprüche:

1. Verbindung der Formel I

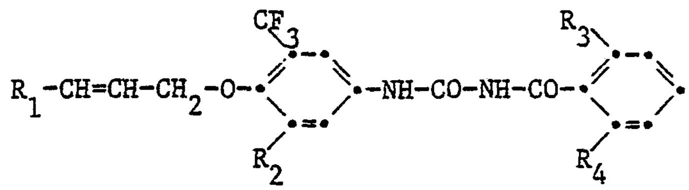

(I) .

worin $R_1$ Wasserstoff, Chlor oder Brom; $R_2$ Chlor oder Brom; $R_3$ Fluor oder Chlor; und $R_4$ Wasserstoff, Fluor oder Chlor bedeuten.

2. Verbindungen der Formel I gemäss Anspruch 1, dadurch gekennzeichnet, dass $R_1$ und $R_2$ Chlor oder Brom; $R_3$ und $R_4$ Fluor bedeuten.

3. Verbindung der Formel 1, gemäss Anspruch 1, dadurch gekennzeichnet, dass $R_1$ und $R_2$ Chlor oder Brom, $R_3$ Chlor und $R_4$ Wasserstoff bedeuten.

4. Verbindung der Formel I gemäss Anspruch 1, dadurch gekennzeichnet, dass $R_1$ Chlor bedeutet.

5. Verbindung gemäss Anspruch 2 der Formel

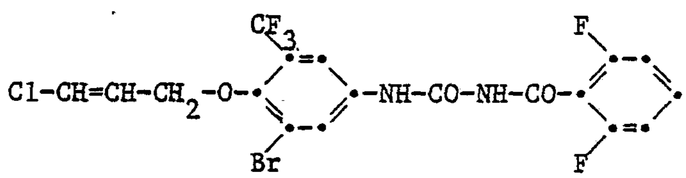

.

6. Verbindung gemäss Anspruch 2 der Formel

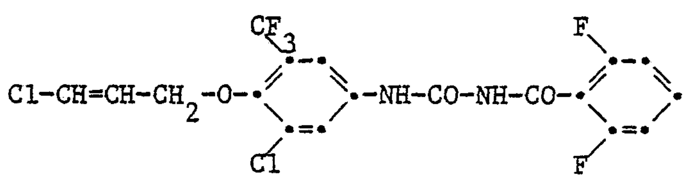

.

7. Verbindung gemäss Anspruch 2 der Formel

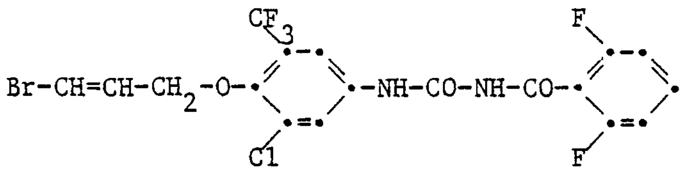

.

8. Verbindung gemäss Anspruch 1 der Formel

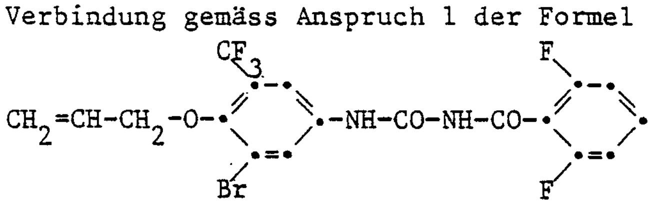

.

9. Verbindung gemäss Anspruch 3 der Formel

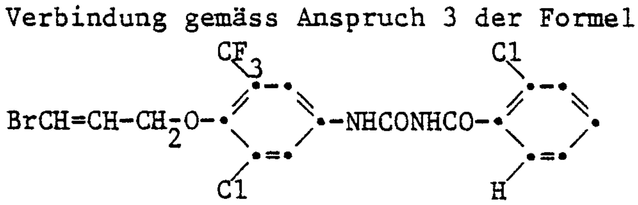

.

10. Verbindung gemäss Anspruch 3 der Formel

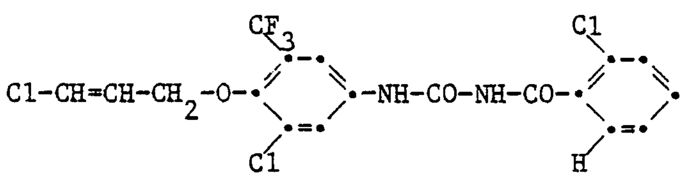

.

11. Verbindung gemäss Anspruch 1 der Formel

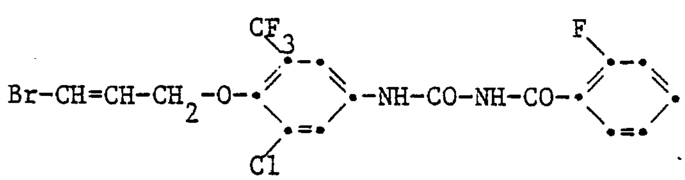

.

12. Verbindung gemäss Anspruch 1 der Formel

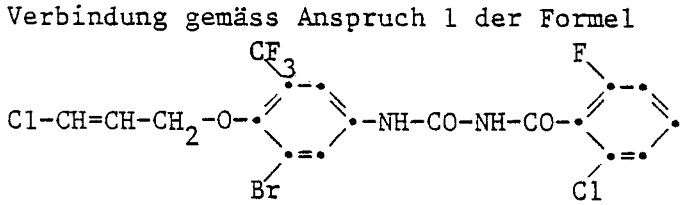

.

13. Verfahren zur Herstellung einer Verbindung gemäss den Ansprüchen 1 bis 12, dadurch gekennzeichnet, dass man

a) eine Verbindung der Formel II

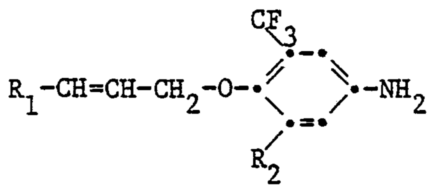

(II)

mit einer Verbindung der Formel III

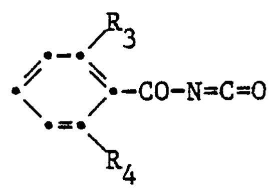

(III)

oder

b) eine Verbindung der Formel IV

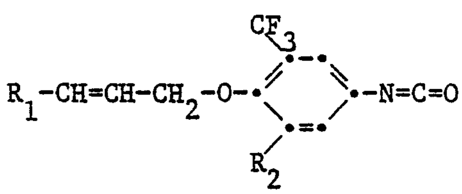

(IV)

mit einer Verbindung der Formel V

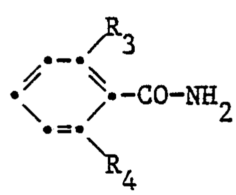

(V)

umsetzt, wobei in den Formeln II bis V die Reste $R_1$, $R_2$, $R_3$ und $R_4$ die in den Ansprüchen 1 bis 4 angegebenen Bedeutungen haben.

14. Schädlingsbekämpfungsmittel, welches als aktive Komponente eine Verbindung gemäss den Ansprüchen 1 bis 12 zusammen mit geeigneten Trägern und/oder anderen Zuschlagstoffen enthält.

15. Verwendung einer Verbindung gemäss den Ansprüchen 1 bis 12 zur Bekämpfung von Schädlingen, vorzugsweise von Insekten.

16. Verwendung gemäss Anspruch 15 zur Bekämpfung larvaler Stadien von Insekten.

0030518

Europäisches Patentamt

# EUROPÄISCHER RECHERCHENBERICHT

Nummer der Anmeldung
EP 80 81 0334

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | EP - A - 0 001 203 (CIBA-GEIGY)<br>* Ansprüche *<br>--<br>EP - A - 0 004 030 (CIBA-GEIGY)<br>* Ansprüche *<br>---- | 1-16<br><br><br>1-16 | C 07 C 127/22<br>A 01 N 47/34 |

RECHERCHIERTE SACHGEBIETE (Int. Cl.³)

C 07 C 127/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 13-02-1981 | GAUTIER |

EPA form 1503.1 06.78